Europäisches Patentamt

European Patent Office

Office européen des brevets

(11) Publication number: **0 450 573 A2**

# EUROPEAN PATENT APPLICATION

(21) Application number: 91105194.4

(22) Date of filing: 02.04.91

(51) Int. Cl.⁵: **C12P 21/08**, A61K 39/395, C12N 5/16, G01N 33/569, G01N 33/577

(30) Priority: 06.04.90 US 505409

(43) Date of publication of application:
09.10.91 Bulletin 91/41

(84) Designated Contracting States:
AT BE CH DE DK ES FR GB GR IT LI LU NL SE

(71) Applicant: **ONCOGEN LIMITED PARTNERSHIP**
**3005 First Avenue**
**Seattle, WA 98121(US)**

(72) Inventor: **Darveau, Richard P.**
**12914 N.E. 1011th Place**
**Kirkland, Washington 98033(US)**

(74) Representative: **Kinzebach, Werner, Dr. et al**
**Patentanwälte Reitstötter, Kinzebach und**
**Partner Sternwartstrasse 4 Postfach 86 06 49**
**W-8000 München 86(DE)**

(54) Antibodies for the treatment and diagnosis of pseudomonas aeruginosa infections.

(57) Antibodies are provided which are useful for the diagnosis, treatment or prevention of P. aeruginosa infection. The antigen to which these antibodies bind contains an epitope recognized by monoclonal antibody 6D7 or 4A12.

EP 0 450 573 A2

The present invention relates to the treatment and diagnosis of bacterial infections, and to novel materials used therein. More particularly, it relates to the production and use of antibodies which react immunologically with an antigen in bacteria, and which in combination with normally suboptimal levels of antibiotic have a bactericidal effect.

Diseases caused by gram-negative bacteria result in significant morbidity and mortality in human patients. This is particularly true of the gram-negative organism Pseudomonas aeruginosa (P. aeruginosa), which has been increasingly associated with nosocomial infections. These infections are encountered very frequently in certain susceptible groups of patients, for example, granulocytopenic patients, surgery patients, patients with trauma involving internal injuries, and patients in intensive care units, especially those on respirators. Infections with P. aeruginosa are frequently encountered in compromised hosts, especially in patients with cystic fibrosis, thermal burns, and cancer.

The therapy of choice for controlling diseases caused by gram-negative organisms has been treatment with antibiotics. However, the continued high morbidity and mortality associated with these diseases is indicative of the limitations of antibiotic therapy. See, for example, EORTC International Antimicrobial Therapy Cooperative Group (1987), N. Engl. J. Med. 317:1692-1698, and Andriole, V.G. (1978), J. Lab. Clin. Med. 94, 196-199. Hence there is a need for alternative therapies with greater effectiveness than treatment solely with antibiotics.

Several types of alternative therapies for infections caused by gram negative bacteria, and P. aeruginosa in particular, have been explored. One form of alternative therapy is augmentation of the host's immune system by active or passive immunization. For example, it has been shown that active immunization of humans or experimental animals with whole cell bacterial vaccines or with purified bacterial endotoxins from P. aeruginosa causes the production of opsonic antibodies directed primarily against determinants on the repeating oligosaccharide units of lipopolysaccharide (LPS) molecules of the outer cell membrane of P. aeruginosa (see Pollack (1979) in Immunoglobulins: Characteristics and Uses of Intravenous Preparations (Alving and Finlayson, eds. pp. 73-79, U.S. Department of Health and Human Services). These antibodies are reportedly protective against P. aeruginosa in some animal models (Pollack, supra), and preliminary results suggest that they may also be protective in humans (Young and Pollack (1980), Pseudomonas Aeruginosa, Sabath, L., ed., pp. 119-132, Hans Huber, 1980). Pennington et al., also, report that intravenous immune globulin ("IVIG") treatment of necrotizing pneumonia caused by P. aeruginosa in a murine model enhanced survival compared to an untreated group (Pennington, Pier, and Small (1986), J. Crit. Care 1:4-10).

It has been suggested that immunotherapeutic approaches could be utilized to prevent and treat bacterial disease due to P. aeruginosa. One approach is to administer pooled human immune globulins that contain antibodies against the infecting strain(s) (see, for example, Collins and Roby (1984), Am. J. Med., 76(3A):168-174). This treatment, however, has certain limitations. For example, the immune globulin compositions consist of pools of samples from many donors, the samples being preselected for the presence of particular anti-Pseudomonas antibodies. The resultant pooled samples may have considerable variability in the titers of the desired antibodies. In addition, the preselection process is expensive. Moreover, products derived from human serum often contain other pathologic substances, e.g., viruses which infect humans, such as the virus(es) which cause acquired immunodeficiency syndrome (AIDS), and the various viruses which cause hepatitis.

Other suggested alternative forms of treatment utilize monoclonal antibodies which react immunologically with gram negative bacteria, and particularly with P. aeruginosa. A variety of monoclonal antibodies directed towards P. aeruginosa have been isolated which are reportedly protective against P. aeruginosa infections. These include single and multi-serotype specific surface epitopes, such as those found in LPS molecules of the bacteria. In addition, purportedly protective monoclonal antibodies specific for exotoxin A have been produced. Other types of monoclonal antibodies which are directed towards epitopes on the flagellar proteins of P. aeruginosa have been described in U.S. Patent No. 4,834,976, issued May 30, 1989. However, for complete treatment or diagnosis, two monoclonal antibodies may be required to react immunologically with organisms bearing type a and type b flagella.

Since the serotype of infecting strains often is not known when therapeutic treatment of the infected individual must begin, it would be desirable to have a monoclonal antibody which offers broad protection capability. In addition, it would be desirable if this antibody increased the effectiveness of antibiotic treatment.

EPA Publication Number 256,713 discloses hybridomas and human lymphoblastoid cell lines which secrete antibodies which reportedly are reactive with lipopolysaccharide that is present on the surface of P. aeruginosa.

U.S. Patent No. 4,834,976 discloses monoclonal antibodies which reportedly react immunologically with

2

P. aeruginosa flagella.

U.S. Patent No. 4,772,465 discloses a method for treating individuals with burn wound sepsis. The method comprises administering ciprofloxacin to attain high concentrations, and also administering Pseudomonas immune globulin.

U.S. Patent No. 4,777,136 discloses a hybridoma cell line which produces monoclonal antibodies that are reportedly reactive with an antigenic determinant of lipopolysaccharide which is common to all strains of P. aeruginosa and to P. maltophilia, and which is not reactive substantially with any antigenic determinant of other clinically important gram negative bacteria. The antibodies bind to a component with an apparent molecular weight of approximately 12 Kd.

U.S. Patent No. 4,834,975 discloses human lymphocyte cell lines which reportedly secrete human monoclonal antibodies to serotypic determinants on LPS molecules of P. aeruginosa.

Since treatment for infections by gram-negative organisms is often required before the organisms causative of the disease are identified, the initial therapy is usually treatment with antibiotics. However, this type of therapy is often ineffective because the concentration of antibiotic which reaches the infection site(s) is below that required to kill the microorganism and/or limit its replication. Moreover, since antibiotics are often toxic as well as expensive, increasing the dosage to achieve increased effectiveness is often not possible. This problem is overcome by the present invention, which provides antibodies which exhibit broad immunological reactivity with P. aeruginosa strains, and whose bactericidal activity is enhanced by sub-inhibitory concentrations of antibiotics. These antibodies react immunologically with a P. aeruginosa antigen, which contains epitopes recognized by monoclonal antibodies 6D7 and/or 4A12.

Monoclonal antibodies 6D7 and 4A12 are the immunologic products of mice which had been immunized with different strains of P. aeruginosa which, prior to immunization, had been incubated in the presence of sub-inhibitory concentrations of antibiotic. Studies with these monoclonal antibodies yielded the surprising results that although their immunological reactivity was specific for P. aeruginosa, it was broad with respect to the P. aeruginosa strains, and that the bactericidal activity of the monoclonal antibodies was enhanced by sub-inhibitory concentrations of antibiotics. Studies characterizing the antigen which contains the epitopes to which these antibodies are immunologically reactive indicate that the antigen is one which has been heretofore unidentified.

As a result of their broad specificity for P. aeruginosa strains, and/or their enhanced killing activity in the presence of low levels of antibiotics, the antibodies of the invention are useful for both the treatment and diagnosis of P. aeruginosa infections.

Accordingly, one aspect of the invention is a composition comprising a first antibody which is capable of immunologically reacting with an antigen with which a second antibody is capable of immunologically reacting, wherein the second antibody is selected from the group of monoclonal antibodies produced by hybridoma 6D7 and hybridoma 4A12, and wherein the composition is free of naturally occurring antibodies which react with other P. aeruginosa antigens.

Another aspect of the invention is a composition comprising a first monoclonal antibody or binding fragment thereof which is capable of immunologically reacting with an antigen with which a second monoclonal antibody is capable of immunologically reacting, wherein the second monoclonal antibody is selected from the group of monoclonal antibodies produced by hybridoma 6D7 and hybridoma 4A12.

Still another aspect of the invention is a cell line which produces a monoclonal antibody capable of immunologically reacting with an antigen with which a monoclonal antibody selected from the group of monoclonal antibodies produced by hybridoma 6D7 and hybridoma 4A12 is capable of immunologically reacting.

Yet another aspect of the invention is a cell line having the identifying characteristics of a cell line selected from the group consisting of hybridoma 4A12 and hybridoma 6D7.

Another aspect of the invention is a method for producing a hybridoma which produces monoclonal antibodies which are capable of immunologically reacting with an antigen with which a second monoclonal antibody is capable of immunologically reacting, wherein the second monoclonal antibody is selected from the group of monoclonal antibodies produced by hybridoma 6D7 and hybridoma 4A12, comprising:

a. immunizing an individual with P. aeruginosa;

b. immortalizing antibody producing cells from the immunized individual;

c. selecting an immortal cell which produces antibodies which react immunologically with the antigen to which monoclonal antibodies produced by hybridoma 6D7 or hybridoma 4A12 react immunologically; and

d. growing said immortal cells.

Still another aspect of the invention is a method for producing a hybridoma which produces monoclonal antibodies which are capable of immunologically reacting with an antigen with which a second monoclonal antibody is capable of immunologically reacting, wherein the second monoclonal antibody is selected from the group of monoclonal antibodies produced by hybridoma 6P7 and hybridoma 4A12, comprising:

a. immunizing an individual with P. aeruginosa, wherein the P. aeruginosa has been treated with a subinhibitory level of antibiotic prior to the immunizing;

b. immortalizing antibody producing cells from the immunized individual;

c. selecting cells which produce monoclonal antibodies which bind immunologically to at least two strains of P. aeruginosa other than the immunizing strain, whether or not these other strains were grown in the presence of antibiotic; and

d. selecting a cell which produces monoclonal antibodies which exhibit enhanced killing of P. aeruginosa cells due to subinhibitory levels of antibiotic, when the P. aeruginosa cells are incubated in the presence of complement, polymorphonuclear cells, and said antibodies; and

e. culturing said cell.

Fig. 1 is a graph showing the results of the effect of antibody concentration on the opsonophagocytic assay with P. aeruginosa using monoclonal antibody 6D7.

Fig. 2 is a half-tone copy of a photograph of an immunoblot of P. aeruginosa whole cell lysates separated on 12% polyacrylamide gel, using monoclonal antibody 6D7.

Fig. 3 is a half-tone copy of a photograph of an immunoblot of P. aeruginosa whole cell lysates separated on 12% polyacrylamide gel, using monoclonal antibody 6D7 (lane 3) and monoclonal antibody 4A12 (lane 4).

Fig. 4 is a half-tone copy of a photograph of immunoblotted whole cell lysates of P. aeruginosa FI-6, separated on a 4-20% SDS polyacrylamide gel, using monoclonal antibodies directed to FI-1 LPS, FI-6 LPS, and monoclonal antibodies "MAb6D7" and "MAb4A12", produced by hybridoma cell lines 6D7 and 4A12, respectively.

Fig. 5 is a half-tone copy of a photograph of an immunoblot of P. aeruginosa whole cell lysates separated on a 4 to 12% polyacrylamide gradient gel, using monoclonal antibody 6D7.

Fig. 6 is a half-tone copy of a photograph of an immunoblot of an outer membrane fraction of P. aeruginosa strain AK1401, using monoclonal antibodies 6D7 and N1F10.

The present invention provides a significant advance in the art of the treatment and diagnosis of infections due to gram negative bacteria, in particular, to infections caused by P. aeruginosa. Monoclonal antibodies have been prepared which each exhibit broad immunological reactivity with P. aeruginosa strains, and whose bactericidal activities are enhanced by subinhibitory concentrations of antibiotics. These antibodies, 6D7 and 4A12, bind immunologically to epitopes on a hitherto unidentified P. aeruginosa antigen. Antibodies which react immunologically with the antigen(s) with which 6D7 and 4A12 react are expected to exhibit the characteristics of those two antibodies with respect to the broad strain specificity, and with respect to an enhanced bactericidal activity in the presence of subinhibitory concentrations of antibiotics which are known by those of skill in the art to be effective against P. aeruginosa.

As used herein, "epitope" or "antigenic determinant" refers to a site on an antigenic molecule that is immunogenic (i.e., which induces an immune reaction), and to which a specific antibody molecule subsequently binds. Fragmentation of antigens by limited proteolysis reveals that individual antigens may possess several or many such determinant sites or epitopes, depending upon the molecular size and complexity of tertiary structure. Methods of determining the spatial conformation of amino acids are known in the art, and include, for example, x-ray crystallography and 2-dimensional nuclear magnetic resonance.

An antigen is "immunologically reactive" with an antibody when it binds to an antibody due to antibody recognition of a specific epitope contained within the antigen. Immunological reactivity may be determined by antibody binding, more particularly by the kinetics of antibody binding, and/or by competition in binding using as competitor(s) a known antigen containing an epitope against which the antibody is directed. The techniques for determining whether an antigen is immunologically reactive with an antibody are known in the art.

As used herein, the term "antibody" refers to a polypeptide or group of polypeptides which are comprised of at least one antibody combining site. An "antibody combining site" or "binding domain" is formed from the folding of variable domains of an antibody molecule(s) to form three-dimensional binding spaces with an internal surface shape and charge distribution complementary to the features of an epitope of an antigen, which allows an immunological reaction with the antigen. An antibody combining site may be formed from a heavy and/or a light chain domain ($V_H$ and $V_L$, respectively), which form hypervariable loops which contribute to antigen binding. The term "antibody" includes, for example, vertebrate antibodies, hybrid antibodies, chimeric antibodies, altered antibodies, univalent antibodies, the Fab proteins, and single domain antibodies.

As used herein, a "single domain antibody" (dAB) is an antibody which is comprised of a $V_H$ domain and reacts immunologically with a designated antigen. A dAB does not contain a $V_L$ domain, but may contain other antigen binding domains known to exist in antibodies, for example, the kappa and lambda domains. Methods for preparing dABs are known in the art. See, for example, Ward et al. (1989).

Antibodies may also be comprised of $V_H$ and $V_L$ domains, as well as other known antigen binding domains. Examples of these types of antibodies and methods for their preparation are known in the art (see, e.g., U.S. Patent No. 4,816,467, which is incorporated herein by reference), and include the following. For example, "vertebrate antibodies" refers to antibodies which are tetramers or aggregates thereof, comprising light and heavy chains which are usually aggregated in a "Y" configuration and which may or may not have covalent linkages between the chains. In vertebrate antibodies, the amino acid sequences of all the chains of a particular antibody are homologous with the chains found in one antibody produced by the lymphocyte which produces that antibody in situ, or in vitro (for example, in hybridomas). Vertebrate antibodies typically include native antibodies, for example, purified polyclonal antibodies and monoclonal antibodies. Examples of the methods for the preparation of these antibodies are described infra.

"Hybrid antibodies" are antibodies wherein one pair of heavy and light chains is homologous to those in a first antibody, while the other pair of heavy and light chains is homologous to those in a different second antibody. Typically, each of these two pairs will bind different epitopes, particularly on different antigens. This results in the property of "divalence", i.e., the ability to bind two antigens simultaneously. Such hybrids may also be formed using chimeric chains, as set forth below.

"Chimeric antibodies" are antibodies in which the heavy and/or light chains are fusion proteins. Typically, the constant domain of the chains is from one particular species and/or class, and the variable domains are from a different species and/or class. Also included is any antibody in which either or both of the heavy or light chains are composed of combinations of sequences mimicking the sequences in antibodies of different sources, whether these sources be differing classes, or different species of origin, and whether or not the fusion point is at the variable/constant boundary. Thus, it is possible to produce antibodies in which neither the constant nor the variable region mimic known antibody sequences. It then becomes possible, for example, to construct antibodies whose variable region has a higher specific affinity for a particular antigen, or whose constant region can elicit enhanced complement fixation, or to make other improvements in properties possessed by a particular constant region.

Another example is "altered antibodies," which refers to antibodies in which the naturally occurring amino acid sequence in a vertebrate antibody has been varied. Utilizing recombinant DNA techniques, antibodies can be redesigned to obtain desired characteristics. The possible variations are many, and range from the changing of one or more amino acids to the complete redesign of a region, for example, the constant region. Changes in the constant region, in general, are to attain desired cellular process characteristics, e.g., changes in complement fixation, interaction with membranes, and other effector functions. Changes in the variable region may be made to alter antigen binding characteristics. The antibody may also be engineered to aid the specific delivery of a molecule or substance to a specific cell or tissue site. The desired alterations may be made by known techniques in molecular biology, e.g., recombinant techniques, site directed mutagenesis, etc.

Yet another example are "univalent antibodies", which are aggregates comprised of a heavy chain/light chain dimer bound to the Fc (i.e., constant) region of a second heavy chain. This type of antibody escapes antigenic modulation. See, e.g., Glennie et al. (1982).

Included also within the definition of antibodies are "Fab" fragments of antibodies. The "Fab" region refers to those portions of the heavy and light chains which are roughly equivalent, or analogous, to the sequences which comprise the branch portion of the heavy and light chains, and which have been shown to exhibit immunological binding to a specified antigen, but which lack the effector Fc portion. "Fab" includes aggregates of one heavy and one light chain (commonly known as Fab'), as well as tetramers containing the 2H and 2L chains (referred to as $F(ab)'_2$), which are capable of selectively reacting with a designated antigen or antigen family. "Fab" antibodies may be divided into subsets analogous to those described above, i.e, "vertebrate Fab", "hybrid Fab", "chimeric Fab", and "altered Fab". Methods of producing "Fab" fragments of antibodies are known within the art and include, for example, proteolysis, and synthesis by recombinant techniques.

As used herein, the term "immunogenic antigen" is an antigen that elicits a cellular and/or humoral immune response, whether alone or linked to a carrier in the presence or absence of an adjuvant.

"Treatment" as used herein refers to prophylaxis and/or therapy.

An "individual", as used herein, refers to vertebrates, particularly members of the mammalian species, and includes but is not limited to domestic animals, sports animals, and primates, including humans.

As used herein, "antibody containing body component" refers to a component of an individual's body which is a source of the antibodies of interest. Antibody containing body components are known in the art, and include but are not limited to, for example, plasma, serum, spinal fluid, lymph fluid, the external secretions of the respiratory, intestinal, and genitourinary tracts, tears, saliva, milk, white blood cells, and myelomas.

As used herein, a "biological sample" refers to a sample of tissue or fluid isolated from an individual, including but not limited to, for example, plasma, serum, spinal fluid, lymph fluid, the external sections of the skin, respiratory, intestinal, and genitourinary tracts, tears, saliva, milk, blood cells, tumors, organs, and also samples of in vitro cell culture constituents (including but not limited to, conditioned medium resulting from the growth of cells in cell culture medium, putatively infected cells, recombinant cells, and cell components).

The procedures for preparing hybridomas 6D7 and 4A12 are provided below in the Examples. The antibodies produced by these cells may be used to isolate and purify the antigen(s) which contain the epitopes to which they bind. For example, cell lysates of P. aeruginosa, prepared by methods known in the art, may be used as a source of antigen. These lysates may be subjected to methods known for the purification of antigens, including, for example, column chromatography, electrophoresis on gels, and immunoaffinity chromatography. Fractions containing the antigen(s) are detectable by monoclonal antibodies 6D7 and 4A12. In addition, these antibodies may be fixed to a solid substrate using techniques known in the art, and used for immunoaffinity chromatography of the antigen. The studies reported infra suggest that the antigen is heat stable, and that the epitopes recognized by 6D7 and 4A12 are insensitive to proteolysis. In addition, the studies show that the antigen is distinguishable from A Band and B Band fractions of lipopolysaccharide (LPS).

Although the procedures described in the Examples for the production of hybridomas 6D7 and 4A12 may be used to obtain other hybridomas which produce antibodies which are similar to 6D7 and 4A12, alternative procedures may also be used to obtain cells which produce antibodies that immunologically react with the same antigen(s) and that also exhibit enhanced bactericidal effects at suboptimal levels of antibiotics. For example, these cells may be produced from antigen-stimulated lymphocytes obtained from individuals which have been immunized with different strains of P. aeruginosa, preferably with bacteria which had been previously treated with subinhibitory concentrations of an antibiotic to which this type of gram-negative bacteria is known to be sensitive. Immortal, antibody-secreting cell lines are then produced by techniques known in the art, including, for example, by fusion with myeloma cells or other fusion partners capable of replicating indefinitely in cell culture, by transformation with EB-virus, or with oncogenic DNA. Techniques for producing hybridomas are discussed in, for example, Hammerling et al. (1981), MONOCLONAL ANTIBODIES AND T-CELL HYBRIDOMAS; See also, U.S. Patent Nos. 4,341,761; 4,399,121; 4,427,783; 4,444,887; 4,464,465; 4,466,917; 4,472,500; 4,491,632; and 4,493,890.

The antibody-producing immortal cells are then screened for the production of antibodies which immunologically react with the antigen(s) which contain the epitopes recognized by monoclonal antibodies 6D7 and 4A12. Methods for screening for cross-immunological reactivity between antibodies are known in the art. For example, the binding of the antibodies may be compared using immunoblot assays of P. aeruginosa cell lysates (see infra). Alternatively, for example, if the epitopes recognized by the antibodies being screened by 6D7 and/or 4A12 are in relatively close proximity or are identical, it may be possible to screen for the desired antibody-producing cells using antibody competition assays.

In addition to the immunological cross-reactivity with monoclonal antibodies 6D7 and 4A12, the antibodies produced by other hybridomas may be screened for the enhanced bactericidal effect in the presence of subinhibitory concentrations of antibiotics which are effective against P. aeruginosa. Antibiotics which are effective in preventing P. aeruginosa replication are known in the art (see, for example, REMINGTON'S PHARMACEUTICAL SCIENCES, 17th Edition, Mack Publishing Company, 1985). Included among these antibiotics are, for example, aminoglycosides (e.g., amikacin, gentamycin, tobramycin, netilmicin, and sisomicin); cephalosporins (e.g., cefepime), particularly third generation cephalosporins (e.g., cefoperazone, cefmenoxime, cefsulodin, ceftazidime, ceftizoxime, ceftriaxone, and moxalactam); penicillins, particularly third and fourth generation penicillins (e.g., carbenicillin, ticarcillin, indanylcarbenicillin, azlocillin, mezlocillin, and piperacillin); and tetracyclines. Methods for determining which antibiotics are effective in preventing replication of P. aeruginosa are known in the art.

An alternative to the above method of producing antibodies which cross-react with the antigen with which 6D7 and 4A12 react employs the purified antigen as an immunogen. As discussed above, the monoclonal antibodies produced by hybridomas 6D7 and 4A12 can be readily employed to isolate the antigen.

The desired antibodies may also be purified from populations of polyclonal antibodies. The polyclonal antibodies may be formed by immunization of an individual with P. aeruginosa, preferably with antibiotic treated P. aeruginosa, and/or with the isolated antigen(s) which bind to 6D7 and/or 4A12. The desired antibodies may then be purified by techniques known in the art, including immunoaffinity chromatography using monoclonal antibodies 6D7 and/or 4A12.

Moreover, a variety of antibodies may be designed using the genetic sequences in hybridomas 6D7 and/or 4A12 which encodes the antibodies or the desired fragments thereof. These various antibodies include, for example, hybrid antibodies, chimeric antibodies, altered antibodies, univalent antibodies, the Fab proteins, and single domain antibodies. Techniques for obtaining the genetic sequences encoding a desired polypeptide, including an antibody, are known in the art, as are methods for their cloning, and insertion into expression vectors. Usually the segment cloned will encode a sufficient portion of the antibody binding domain(s) so that immunological binding to the antigen which contains the 6D7 and/or 4A12 epitopes will be retained; however, the avidity of the binding may be altered in the recombinantly produced molecule. The desired antibodies may then be produced by their expression from cells (either prokaryotic or eukaryotic). Alternatively, using the information from the nucleotide sequence, the antibodies may be produced by chemical synthetic methods, which are known in the art.

The antibodies of the invention may be used for treatment of P. aeruginosa infections, including septic shock. The antibodies are administered in effective amounts, either intravenously or intramuscularly in a physiologically acceptable solution. Multiple doses of the antibodies may be given, as is needed for effectiveness. The administration of the antibodies is preferably in conjunction with antibiotics in an amount that will enhance the bactericidal effect of the antibiotics on P. aeruginosa. The administration of the antibiotics may be prior to and/or simultaneous with, and/or after the administration of the antibodies. Because the antibodies have a selective affinity for P. aeruginosa, they provide selective treatment for such infections, which are otherwise often unresponsive to antibiotic treatment.

The antibodies of the invention may also be modified to be complexed with/or contain an effector which is bactericidal for targeted cells. The effector is a substance which is toxic to P. aeruginosa and which is known in the art, including, for example, inhibitors of bacterial replication such as antibiotics, antibacterials, anti-sense RNA, or radioisotopes emitting "hard", e.g., beta radiation. Radioisotopes which emit "hard" radiation include, for example, yttrium-90, phosphorus-32, lead-212, iodine-131, and palladium-109. In some cases, the effector may be entrapped in a delivery system such as a liposome or dextran carrier.

The antibodies of the invention may also be used prophylactically in patients at risk for P. aeruginosa infection. Administration of effective amounts of these monoclonal antibodies, in conjunction with antibiotics, serves to enhance the body's potential ability to defend against these organisms, thereby lessening the risk of subsequent infection.

The antibodies of the invention may also be used in vitro as diagnostic agents to test for the presence of P. aeruginosa in an individual by subjecting biological sample(s) from the individual to the antibodies using standard immunoassay protocols. Additionally, extracts of inanimate objects of which contamination by P. aeruginosa contamination would be detrimental, such as medical devices, foodstuffs or water, may also be tested. The design of the immunoassays is subject to a great deal of variation, and many formats are known in the art. The immunoassay will use an antibody directed against the antigen(s) which contain the epitope(s) recognized by monoclonal antibody(s) 6D7 and/or 4A12. Protocols may be based, for example, upon competition, or direct reaction, or sandwich type assays. Protocols may also, for example, use solid supports, or may be by immunoprecipitation. Most assays involve the use of labeled antibody or polypeptide; the labels may be, for example, enzymatic, fluorescent, chemiluminescent, radioactive, or dye molecules. Assays which amplify the signals from the probe are also known; examples of which include assays which utilize biotin and avidin, and enzyme-labeled and enzyme-mediated immunoassays, such as ELISA assays.

Typically, an immunoassay for P. aeruginosa will involve selecting and preparing the test sample suspected of containing the bacterial cells, then incubating it with the antibodies under conditions that allow the formation of antigen-antibody complexes. Various formats can be employed. For example, a "sandwich assay" may be employed, where antibody bound to a solid support is incubated with the test sample; washed; incubated with a second, labeled antibody to the analyte, and the support is washed again. Analyte is detected by determining if the second antibody is bound to the support. In a competitive format, which can be either heterogeneous or homogeneous, a test sample is usually incubated with antibody and a labeled, competing antigen is also incubated, either sequentially or simultaneously. These and other formats are well known in the art.

Complexes formed comprising the antibodies of the invention (or, in the case of competitive assays, the amount of competing antibody) are detected by any of a number of known techniques, depending on the format. For example, unlabeled antibodies in the complex may be detected using a conjugate of antixenogeneic Ig complexed with a label, (e.g., an enzyme label).

The antibodies of the invention are useful for the in vivo detection of localized areas of P. aeruginosa

7

infection, for example, abscesses or cysts in the soft tissue and osteomyelitis in bone. For such determination, the antibodies, which are labeled, are administered to an individual suspected of having a P. aeruginosa infection. The antibody will selectively bind to these bacteria if present, thereby concentrating the label in the area of infection. Labels appropriate for such use are known in the art, and include, for example, radioisotopes (e.g., [125]Iodine, [131]Iodine, [99]Technetium and [111]Indium), which can be detected using known techniques. Alternatively, for example, the monoclonal antibodies may be labeled with paramagnetic contrast agents, and detected by nuclear magnetic resonance methods.

Kits suitable for immunodiagnosis and therapeutic use, containing the appropriate reagents are constructed by packaging the appropriate materials, including the antibodies of the invention in suitable containers, along with the remaining reagents and materials required for the conduct of the assay or therapy, as well as a suitable set of instructions.

Described below are examples of the present invention which are provided only for illustrative purposes, and not to limit the scope of the present invention. In light of the present disclosure, numerous embodiments within the scope of the claims will be apparent to those of ordinary skill in the art.

## Examples

### Generation and Selection of Hybridoma Cell Line 4A12

Murine hybridoma cell line 4A12 produces a murine monoclonal antibody (MAb) that binds to a variety of different strains of P. aeruginosa, and which exhibits enhanced killing when low levels (i.e., subinhibitory concentrations) of antibiotic are present. This cell line was generated by immunizing mice initially with bacteria from a P. aeruginosa strain, Fisher immunotype 4 (FI-4), which had been isolated from an infected individual's blood, and then with P. aeruginosa strain Fisher immunotype 2 (FI-2). All preparations of immunizing bacteria had been incubated with low levels of an aminoglycoside antibiotic, amikacin, prior to use as an immunogen. The concentration of amikacin was 4 $\mu$g/ml, which is 1/4 the minimum inhibitory concentration ("MIC"). On day 49, polyclonal sera from the immunized mice were examined in ELISA binding assays using two immunologically distinct strains of P. aeruginosa, FI-5 and FI-6, grown in absence of, or in the presence of low levels (1/4 MIC) of either of the antibiotics, cefepime or amikacin. A mouse producing polyclonal sera with a high cross reactive titer on both strains was then injected intravascularly with strain Fisher immunotype 2. Three days following this immunization, the spleen was removed from this mouse, and hybridomas from the spleen cells were prepared essentially as described in U.S. Patent No. 4,834,976, with the exception that mouse myeloma cell line P3XAg8.653 (American Type Culture Collection, "ATCC", 12301 Parklawn Drive, Rockville, MD 20852, Accession No. CRL 1580) was used as the fusion partner. The immunization schedule for the above described procedure is shown in Table 1; bleedings to determine the immune response and for polyclonal analysis were on days 21, 35, and 49.

Table 1

| Injection No. | Day | No. of Cells | Immunizing Strain |
|---------------|-----|--------------|-------------------|
| 1 | 1 | $2.0 \times 10^5$ | FI-4 |
| 2 | 7 | $3.4 \times 10^5$ | FI-4 |
| 3 | 14 | $3.7 \times 10^5$ | FI-4 |
| 4 | 28 | $2.1 \times 10^5$ | FI-4 |
| 5 | 42 | $4.2 \times 10^5$ | FI-4 |
| 6 | 62 | $3.6 \times 10^5$ | FI-2 |

The culture supernatants of the fused cells were assayed for the presence of anti-Pseudomonas antibodies by ELISA on day 10 post-fusion essentially as described in U.S. Patent No. 4,834,976, except that in order to select hybridomas which produced antibodies that were immunologically reactive with other immunologically distinct strains of P. aeruginosa which had been altered by antibiotic, two sequential binding assays were used. The first binding assay detected antibody binding to a strain of P. aeruginosa other than the strains used to immunize the mice, i.e., Fisher immunotype 6 (FI-6). The second binding assay retested binding to the bacterial strain used in the first binding assay, and also to yet another P. aeruginosa strain which differed from the immunizing strain, i.e., Fisher immunotype 5 (FI-5). These assays were accomplished as follows.

8

The antigen plates for the ELISA were prepared by immobilizing viable bacteria in the wells of 96-well microtiter plates, as follows. Fifty $\mu l$ poly-L-lysine ("PLL") (1 $\mu g$/ml in phosphate buffered saline ["PBS"]) were added to each well of 96-well plates and incubated for 30 minutes at room temperature. Unadsorbed PLL was removed, and the wells were washed three times with PBS. Bacterial cultures grown to mid-log phase in Trypticase soy broth ("TSB") containing either no antibiotic, or low levels (1/4 MIC) of cefepime or amikacin, were washed once with PBS and then resuspended in PBS containing appropriate antibiotic to O.D.$_{660}$ = 0.2. Fifty microliters of the bacterial suspensions were added to each well of the plate and allowed to bind at 37°C for one hour. Unbound bacteria in suspension were removed, and the wells were washed three times with saline-Tween (0.9% [w/v] NaCl, 0.05% [v/v] Tween-20).

Nonspecific binding of the antibodies was blocked by the addition of 200 $\mu l$/well of blocking buffer (PBS containing 5% [w/v] non-fat dry milk, 0.01% [v/v] Antifoam A [Sigma, St. Louis, Mo.] and 0.015 [w/v] thimerosal) to the wells, followed by incubation for one hour at room temperature. Excess blocking buffer was removed, and the wells were washed three times with saline-Tween as previously described.

Culture supernatants (50 $\mu l$) were replica plated into the corresponding wells of the assay plates, and incubated at room temperature for 30 minutes. The culture supernatants were removed, and the wells washed five times with saline-Tween.

An enzyme-conjugated second step antibody (horseradish peroxidase-conjugated goat anti-mouse IgG + IgM) (Tago, Inc., Burlingame, Calif.) was diluted in PBS containing 0.1% (v/v) Tween-20 and 0.2% (w/v) BSA according to previously determined titrations, and then 50 $\mu l$ of the reagent was added to each well, and the plates were incubated 30 minutes at room temperature. The excess reagent was removed, the wells washed five times with saline-Tween, and 100 $\mu l$/well of o-phenylenediamine substrate (0.8 mg/ml in 0.1 M citrate buffer, pH 5.0, mixed with an equal volume of 0.03% [v/v] $H_2O_2$) was added. The plates were incubated for 30 minutes at room temperature in the dark, and the reactions were then terminated by the addition of 50 $\mu l$/well of 3N $H_2SO_4$. The presence of bound peroxidase in the wells, which reflects the binding of the mouse monoclonal antibodies, was determined by measuring the $A_{490}$ on a Bio-Tek EL-310 Automated EIA Plate reader.

Using the above-described methods, the culture supernatants from the fused cells were assayed for the presence of antibodies that bound to FI-6 (ATCC No. 27317) which had been grown with low levels (1/4 MIC) of cefepime or amikacin. Supernatants containing antibodies which bound to antibiotic treated FI-6 were then subjected to a second ELISA assay similar to the above described assay, except that the plates contained the following P. aeruginosa as antigens: (1) FI-6 grown in the absence of antibiotics; (2) FI-6 grown in the presence of cefepime (1/4 MIC); (3) FI-6 grown in the presence of amikacin (1/4 MIC); (4) FI-5 grown in the absence of antibiotics; (5) FI-5 grown in the presence of cefepime (1/4 MIC); and (6) FI-5 grown in the presence of amikacin (1/4 MIC). Control wells contained PLL, but did not contain bacteria.

Cells producing antibodies that bound to both FI-5 and FI-6, whether or not the bacteria were grown in the presence of antibiotic, were transferred into larger tissue culture dishes, and were further analyzed for their ability to elicit bacterial killing in the presence of complement and polymorphonuclear cells.

The selected hybridoma cells were grown to maximize antibody production in media that was free of antibiotics. Culture supernatants were then analyzed for the presence of antibodies which elicit killing of P. aeruginosa strain FI-6 as follows.

Overnight cultures of P. aeruginosa FI-6 in adjusted Miller-Hinton broth containing 50 mg/l calcium chloride and 25 mg/l magnesium chloride were innoculated into media lacking or containing 1 $\mu g$/ml amikacin; this concentration of antibiotic allows almost normal growth of the bacterial culture. The bacterial cultures were incubated until mid-logarithmic phase of growth was attained, and then the bacteria were harvested by centrifugation, and resuspended to 5 x $10^4$ cells/ml in gelatin veronal buffer. Each bacterial suspension was then added to a separate 1.5 ml Eppendorf tube containing: 50 $\mu l$ of hybridoma supernatant; 50 $\mu l$ of preabsorbed serum which served as the source of serum complement (the preabsorption was with the test bacterial strain to remove antibodies to the strain, if any); and 300 $\mu l$ of gelatin veronal buffer containing 2.5 x $10^6$ polymorphonuclear cells. Opsonic activity of antibodies directed against amikacin-treated bacteria was determined in the presence of 1 $\mu g$/ml amikacin. The tubes were gently rotated at 37°C for four hours. The amount of bacterial killing, was determined by plating 25 $\mu l$ from each tube onto solid agar, growing the plated bacteria overnight, and counting the number of colonies on the plate. The following controls were used for each assay sample: a tube containing antibody known to kill the bacterial strain (positive control), and a tube containing an antibody known not to kill the bacterial strain (negative control). The amount of killing for each of the culture supernatants examined was compared to the negative control. Any killing greater than the negative control is due to the antibody present in the culture supernatant rather than the antibiotic alone, since the negative control and the test sample contain the same amount of antibiotic.

9

The results showed that one hybridoma supernatant contained antibodies which, in the presence of complement and polymorphonuclear cells, killed the bacteria grown either in the absence or the presence of low levels of amikacin. However, the killing activity was twenty percent greater on the amikacin-grown cells. The cells from this well, designated 4A12, were minicloned and cloned by standard techniques, essentially as described in U.S. Patent No. 4,834,976 and in Tam et al. (1982), Infect. Immun. 36, 1042. In addition, the clonal status of the cell line was assured by plating cells at a dilution of one cell/well in a 72 well Nunclon plate (Scientific Resource Assoc., Inc. Redmond, Wa.), and visually confirming one cell/well. Those wells that contained one cell were transferred to larger standard 96 well plates for growth and antibody production by standard techniques.

The antibody produced by cell line 4A12 was identified as an IgM using the Mouse Isotyping Kit (American Qualex International Inc., La Mirada, CA). The procedure was performed as described by the manufacturer except that antigen plates were prepared using appropriate strains of whole bacteria on PLL prepared plates (as described supra).

Generation and Selection of Hybridoma 6D7

The procedure for obtaining hybridoma 6D7 was similar to that for obtaining hybridoma 4A12, except that the injections for immunization used only viable bacteria from a P. aeruginosa strain Fisher immunotype 4 (FI-4) which had been isolated from the blood of an infected patient. Prior to injection, the bacteria were incubated with amikacin at 4 µg/ml (1/4 MIC). The injection schedule is indicated in Table 2; bleedings for measurement of immune reaction and for analysis of polyclonal antibodies were on days 25, 38, 68, and 86.

Table 2

| Injection No. | Day | No. of Cells | Immunizing Strain |
|---|---|---|---|
| 1 | 1 | $1.7 \times 10^5$ | FI-4 |
| 2 | 10 | $3.3 \times 10^5$ | FI-4 |
| 3 | 17 | $2.4 \times 10^5$ | FI-4 |
| 4 | 32 | $2.9 \times 10^5$ | FI-4 |
| 5 | 61 | $5.8 \times 10^5$ | FI-4 |
| 6 | 79 | $7.0 \times 10^5$ | FI-4 |
| 7 | 101 | $3.6 \times 10^5$ | FI-4 |

Injection 7 of the organisms was given intravascularly to a mouse producing polyclonal sera with a high cross-reactive titer on two strains of P. aeruginosa, FI-5 and FI-6. Three days after this immunization, the spleen was removed from this mouse and the spleen cells were prepared for hybridization, essentially as described in the preparation of hybridoma 4A12, described supra. Selection and cloning of a hybridoma which produced a type of antibody which interacted with a broad variety of P. aeruginosa strains, and which exhibited enhanced killing in the presence of sub-MIC levels of the antibiotic, amikacin, was carried out using the methods described for the selection and cloning of hybridoma 4A12, supra. This selection yielded a hybridoma which was named 6D7.

The antibody produced by hybridoma 6D7 was identified as an IgG$_3$ by the above described isotyping procedures.

In the selection of hybridomas which yielded 4A12 and 6D7, a total number of 9 fusions were examined. This represented 47 plates at 96 wells/plate for a total of 4512 master wells, of which 49 (1%) displayed confirmed cross-reactive binding on strains FI-5 and FI-6 grown with either amikacin or cefepime. Out of these 49 wells, two wells (6D7 and 4A12) displayed functional killing activity greater than 50% from the negative antibody on these strains. This represents 4% of the confirmed master wells, or 0.04% of the total number of master wells examined.

Purification of 6D7 Antibodies

Antibodies synthesized by hybridoma 6D7 were purified from a large scale culture supernatant by affinity chromatography on immobilized protein-A (Repligen Corporation, Cambridge, MA.) essentially as described by Ey et al. (1978), Immunochemistry 15:429. The antibody eluted as a single symmetrical peak after the addition of 0.1 M citrate buffer, pH 3.5. Fractions containing antibody 6D7 were pooled and dialyzed overnight against PBS containing 1.0 M NaCl, and stored at -70° C.

Specificity of Binding of 6D7 and 4A12 Antibodies

The binding patterns of monoclonal antibodies 6D7 and 4A12 were examined by an enzyme-linked immunosorbent assay (ELISA) using a variety of different bacterial strains.

The sources of the bacterial strains used for the binding assays are as indicated. The Fisher Immunotype (FT) and the International Antigenic Typing Scheme (IATS) strains were obtained from the American Type Culture Collection ("ATCC") and have Accession Nos. 27312-27318 and 33348-33364, respectively. Other strains obtained from the ATCC were: P. nutida (ATCC No. 12633), P. cepacia (ATCC 25416), K. pneumonia (ATCC No. 10273), and S. aureus (ATCC 10832). Dr. Fred Tenover, Veterans Administration Hospital, Seattle, WA, supplied the following strains: P. putrefaciens, P. stutzeri, P. testosteroni, and E. aerogenes. The remainder of the strains examined and their sources (in parenthesis) are as listed below: P. maltophilia (Harborview Medical Center, Seattle, WA.); S. marcescens (Center for Disease Control); E. coli H16 (Walter Reed Institute of Research, Washington, D.C.); S. epidermidis (Group Health, Seattle, WA.) and S. algalactiae (Childrens Orthopaedics Hospital, Seattle, WA.). In addition, E. coli 294 was obtained from Dr. Rafael Martinez, Dept. of Microbiology, UCLA, Los Angeles, CA. The identity of all isolates was confirmed by standard biochemical tests. Strains were stored in 15% glycerol at -70° C.

The degree of cross-reactivity of antibodies 6D7 and 4A12 with the above listed strains was determined by ELISA. The assay employed stationary phase bacteria attached to the solid phase which was previously coated with poly-L-lysine, as described in U.S. patent number 4,834,976. This mild coating procedure allows the detection of antibodies that bind surface structures and are functional in subsequent in vivo protection assays (compare example 1 of U.S. patent No. 4,834,976, to example 2 in Raff et al. (1988), J. Infectious Dis. 157, 118. Representative strains of P. aeruginosa from the seven Fisher Immunotypes as well as seventeen from the IATS were examined. In addition, the binding of these antibodies to a variety of different bacteria were determined.

The results presented in Table 3 show that both 6D7 and 4A12 exhibit high binding activity with all seven Fisher immunotype strains as well as 16 of the 17 International serotypes. In contrast, neither antibody bound any of the fourteen non-Pseudomonas species examined. Thus, it appears that the antigenic determinant recognized by monoclonal antibodies 6D7 and 4A12 is present on a wide variety of P. aeruginosa strains, but not on other bacterial species.

## Table 3

### Enzyme-Linked Immunosorbent Assay of 6D7 and 4A12 Binding to Whole Bacteria

|  | 6D7 | 4A12 |
|---|---|---|
| Pseudomonas aeruginosa Fisher Immunotype 1 | 3 | 3 |
| Pseudomonas aeruginosa Fisher Immunotype 2 | 3 | 3 |
| Pseudomonas aeruginosa Fisher Immunotype 3 | 2.41 | 2.72 |
| Pseudomonas aeruginosa Fisher Immunotype 4 | 3 | 3 |
| Pseudomonas aeruginosa Fisher Immunotype 5 | 3 | 3 |
| Pseudomonas aeruginosa Fisher Immunotype 6 | 3 | 3 |
| Pseudomonas aeruginosa Fisher Immunotype 7 | 3 | 3 |
| Pseudomonas aeruginosa International Type 1 | 3 | 3 |
| Pseudomonas aeruginosa International Type 2 | 3 | 3 |
| Pseudomonas aeruginosa International Type 3 | 3 | 3 |
| Pseudomonas aeruginosa International Type 4 | 3 | 3 |
| Pseudomonas aeruginosa International Type 5 | 3 | 3 |
| Pseudomonas aeruginosa International Type 6 | 3 | 3 |
| Pseudomonas aeruginosa International Type 7 | 3 | 3 |
| Pseudomonas aeruginosa International Type 8 | 3 | 3 |
| Pseudomonas aeruginosa International Type 9 | 3 | 3 |
| Pseudomonas aeruginosa International Type 10 | 3 | 3 |
| Pseudomonas aeruginosa International Type 11 | 2.103 | 2.126 |
| Pseudomonas aeruginosa International Type 12 | 3 | 3 |
| Pseudomonas aeruginosa International Type 13 | 0.08 | 0.136 |
| Pseudomonas aeruginosa International Type 14 | 3 | 3 |
| Pseudomonas aeruginosa International Type 15 | 3 | 3 |
| Pseudomonas aeruginosa International Type 16 | 3 | 3 |
| Pseudomonas aeruginosa International Type 17 | 3 | 3 |
| Pseudomonas maltophilia A521 | 0 | 0.003 |
| Pseudomonas putida F496 | 0.004 | 0.011 |
| Pseudomonas putrefaciens G200 | 0.003 | 0.042 |
| Pseudomonas stutzeri G201 | 0 | 0.061 |
| Pseudomonas testosteroni G197 | 0 | 0 |
| Pseudomonas cepacia G143 | 0.042 | 0.121 |
| Klebsiella pneumoniae | 0.034 | 0.253 |
| Enterobacter aerogenes G438 | 0.015 | 0.111 |
| Serratia marcescens F431 | 0.003 | 0.016 |
| Proteus mirabilis E601 | 0.007 | 0.017 |
| Escherchia coli H16 | 0 | 0 |
| Staphylococcus aureus A577 | 0 | 0.018 |
| Staphylococcus epidermidis E321 | 0.017 | 0.022 |
| Streptococcus Group B 1540 | 0.048 | 0.047 |

Effect of Antibiotic at a Subinhibitory Concentration on the Opsonophagocytic Activity of Monoclonal Antibody 6D7

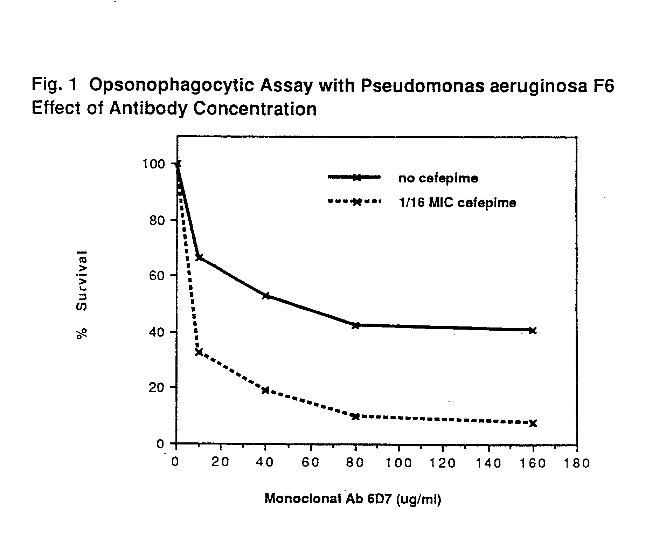

The opsonophagocytic activity of varying amounts of monoclonal antibody 6D7 towards P. aeruginosa strain FI-6 cells was determined in the presence or absence of subinhibitory concentrations of the antibiotic, cefepime. The assay was essentially as described supra, in which hybridomas were screened for the production of monoclonal antibodies which exhibited enhanced opsonic mediated killing in the presence of the antibiotic, amikacin, when the monoclonal antibodies were used in an opsonophagocytic assay. However, cefepime at the concentrations indicated in Figure 1 replaced amikacin. The bacterial cells were grown to mid log phase with the concentration of cefepime used in the assay. In addition, two modifications of the assay were included: (1) the bacteria were incubated with antibody for 30 minutes prior to the addition of complement and polymorphonuclear cells; and (2) $2.5 \times 10^5$ polymorphonuclear cells were used (instead of $2.5 \times 10^6$ cells).

The results of the study, shown in Figure 1, indicate that subinhibitory concentrations of cefepime markedly enhance the opsonophagocytic activity of monoclonal antibody 6D7. The effect of cefepime alone is shown in the 0 μg/ml 6D7 data point where there was 100% survival. Therefore, this data shows that cefepime modifies the bacteria in such a way that the cross-reactive antibody becomes more functional. This bacterial modification by cefepime is absolutely required for enhanced 6D7 activity with P. aeruginosa strain FI-6. Increasing antibody concentration alone is not sufficient to attain maximal activity.

Functional Cross-reactivity of Monoclonal Antibody 6D7

The ability of monoclonal antibody 6D7 ("MAb6D7") to act as an opsonizing agent in the opsonophagocytic assay on a variety of P. aeruginosa strains was examined using essentially the method described supra for the studies on the effect of cefepime in the opsonophagocytic assay. However, as a control the reactions were also carried out with an antibody which does not react immunologically with the strain of P. aeruginosa being examined. For example, when strain FI-6 was examined a monoclonal antibody designated VF5 which reacts with LPS of strain FI-2 was used as a negative control. For examination of strain FI-2, a monoclonal antibody designated IVB2 which is directed against the LPS of FI-6 was used. For strain FI-3, the negative antibody was IVB2, and for examination of strain FI-4, VF5 was the negative control. In addition, the concentrations of cefepime were as indicated in Table 4, where the results of the study are presented. The data in Table 4 on opsonization and subsequent killing in the opsonophagocytic assay due to MAb6D7 is presented as the percent survival of P. aeruginosa cells incubated with MAb6D7 in relation to the bacterial killing with the negative control antibody; the strains examined for bacterial killing were FI-2(A362), FI-3(A363), FI-4(F164), and FI-6(A366). In Table 4, the symbol "nd" indicates that the experiment was not done.

## Table 4

### Functional Cross-reactivity of MAb6D7 in Bactericidal Activity to P. aeruginosa

| | % Survival of P. aeruginosa Strain | | | |
|---|---|---|---|---|
| Cefepime (μg/ml) | FI-2 | FI-3 | FI-4 | FI-6 |
| 0 | 6.6 | 86.9 | 66.9 | 19.7 |
| 0.125 | 5.1 | 108.8 | nd | 5.4 |
| 0.25 | 1.0 | 89.4 | 27.9 | 2.3 |
| 0.5 | 0.27 | 43.1 | 7.4 | 0.5 |
| 1 | nd | nd | 3.5 | 0 |

As seen from the results, MAb6D7 exhibited antibiotic enhanced opsonophagocytic activity with the four P. aeruginosa strains examined. Moreover, the enhancement occurred at relatively low levels of cefepime, i.e., at antibiotic levels which are in the range of 1/8 to 1/32 the minimum inhibitory concentration ("MIC") of the antibiotic.

Functional Cross-reactivity of Monoclonal Antibody 4A12:
The Effect of Cefepime

The ability of monoclonal antibody 4A12 ("MAb4A12") to act as an opsonizing agent in the opsonophagocytic assay on a variety of P. aeruginosa strains was examined using essentially the method described supra for the studies on MAb6D7, except that MAb4A12 replaced MAb6D7. The data in Table 5 on the killing in the opsonophagocytic assay due to MAb4A12 is presented as the percent survival of P. aeruginosa cells incubated with MAb4A12 in relation to the killing with the negative control antibody; the strains examined for opsonophagocytic activity were FI-2(A362), FI-3(A363), FI-4(F164), and FI-6(A366).

## Table 5

### Functional Cross-reactivity of MAb4A12 in Bactericidal
### Activity to P. aeruginosa:  The Effect of Cefepime

#### % Survival of P. aeruginosa Strain

| Cefepime (μg/ml) | FI-2 | FI-3 | FI-4 | FI-6 |
|---|---|---|---|---|
| 0 | 96.6 | 75.7 | 89 | 88.7 |
| 0.125 | 80.8 | 97.6 | nd | 74.6 |
| 0.25 | 80 | 70.5 | 44.1 | 53.6 |
| 0.5 | 46.9 | 74.3 | 27.1 | 54.2 |
| 1.0 | nd | nd | 13.4 | 38.3 |

As seen from the results, MAb4A12 exhibited antibiotic enhanced opsonophagocytic activity with three out of four P. aeruginosa strains examined. Moreover, the enhancement occurred at relatively low levels of cefepime, i.e., at antibiotic levels which are in the range of 1/8 to 1/32 the minimum inhibitory concentration (MIC) of the antibiotic.

Functional Cross-reactivity of Monoclonal Antibody 4A12:
The Effect of Amikacin

The reactivity of MAb4A12 was examined in an opsonophagocytic assay in which the effect of amikacin on the killing effect was examined. The studies were carried out essentially as described above for the studies with cefepime, except that amikacin at the concentrations indicated in Table 6 replaced cefepime, and that the strains examined were FI-1 (A361), FI-6 (A366), and IATS 5 (A374).

## Table 6
### Functional Cross-reactivity of MAb4A12 in Bactericidal
### Activity to P. aeruginosa:  The Effect of Amikacin

| Amikacin (μg/ml) | FI-1(A361) | FI-6(A366) | IATS5(A374) |
|---|---|---|---|
| 0 | 102.5 | 56.5 | 65 |
| 2 | nd | 32.3 | 31 |
| 8 | 60 | nd | nd |

The results in the Table are presented as the percent survival relative to treatment with an antibody which was a negative control, i.e., it didn't bind to the strains indicated. The symbol "nd" indicates that the experiment was not done. As seen in the Table, amikacin stimulated the killing effect with all strains examined. In addition, although strain FI-1(A361) was not killed in the absence of antibiotic, the addition of a relatively low level of amikacin induced killing in the presence of MAb4A12.

In vivo Protective Effect of MAb6D7 in Combination with Antibiotic

The ability of MAb6D7 to be protective against P. aeruginosa infection was examined in the neutropenic mouse model.

Mice were made neutropenic five days prior to bacterial challenge. Neutropenia was induced by subcutaneous injection of 250 mg/kg cytoxan. Four hours prior to bacterial challenge, 100 μg of antibody was injected intraperitoneally in a volume of 0.5ml PBS containing 0.5 M NaCl. All of the antibodies used in the study were purified by protein A Sepharose chromatography. The mice were challenged with either 2 x $10^4$ P. aeruginosa FI-6 (ATCC 27317) or with 1 x $10^4$ P. aeruginosa FI-3. Challenge was by intraperitoneal injection.

In those mice receiving antibiotic, cefepime was administered intramuscularly in two equal doses of 6.25 mg/kg in phosphate-buffered saline (PBS); administration was 1 hour and 3 1/2 hours after the challenge with bacteria. In order to assess the passive protective effect of MAb6D7, the mice were immunized with either MAb6D7, or with a control antibody PA10 VF5, which does not bind to the bacteria used in the challenge. The mice were divided into four groups. One group received MAb6D7 without antibiotic; a second group received MAb6D7 with cefepime; the third and fourth groups received negative control antibody with and without cefepime, respectively. The survival of the mice was monitored for a period of 10 days.

When the protective effect of MAb6D7 and the negative control antibody were compared using strain FI-6, there was a significant increase ($p<0.02$, two sample T test) in the amount of survival with antibody 6D7 (Table 7). In Table 7, the data on protection is presented as the mean ± (one standard deviation); "n" is the number of mice examined for each group. The results indicate that with FI-6, there was some protective effect of the antibody in the absence of cefepime. Of consequence, there was a significant increase ($p<.02$, two sample T test) in the percent survival when low doses of cefepime were coadministered with antibody 6D7. Under the same conditions, there was no significant difference in the percent survival ($p>0.7$, two sample T test) when mice given the negative control antibody with and without cefepime were compared.

## Table 7

### PROTECTION AGAINST P. AERUGINOSA STRAIN FI-6 INFECTION BY MAb6D7

| Treatment | | Percent Survival |
|---|---|---|
| Antibody | Antibiotic | |
| MAb6D7 | None | 36.87 ± (14.37) n=53 |
| MAb6D7 | Cefepime | 63.12 ± (20.16) n=63 |
| PA10 VF5 | None | 16.47 ± (14.35) n=43 |
| PA10 VF5 | Cefepime | 18.75 ± (12.46) n=65 |

The protective effect of monoclonal antibody 6D7 administered with or without cefepime was also examined using strain FI-3. These experiments were performed as described above for strain FI-6, except that 20 mice were examined in each of four groups. The results, presented in Table 8, show that monoclonal antibody 6D7 was unable to protect mice from infection with this strain when cefepime was absent (no significant difference between negative antibody and MAb6D7, $p>.25$, log rank test). However, when cefepime was co-administered with 6D7, there was a significant difference in the survival rate of the mice ($p<.025$, log rank test). No significant difference in the survival rate was observed ($p>.25$, log rank test) when the negative control antibody was administered with and without cefepime.

## Table 8

### PROTECTION AGAINST P. AERUGINOSA STRAIN FI-3 INFECTION BY MAb6D7

| Treatment | | Percent Survival by Day | | | | | | | | | | |
|---|---|---|---|---|---|---|---|---|---|---|---|---|
| Antibody | Antibiotic | 0 | 1 | 2 | 3 | 4 | 5 | 6 | 7 | 8 | 9 | 10 |
| 6D7 | None | 100 | 100 | 20 | 20 | 20 | 20 | 20 | 20 | 20 | 20 | 20 |
| 6D7 | cefepime | 100 | 100 | 60 | 40 | 40 | 40 | 40 | 40 | 40 | 40 | 40 |
| PA10 VF5 | None | 100 | 100 | 30 | 20 | 20 | 20 | 20 | 20 | 20 | 20 | 20 |
| PA10 VF5 | cefepime | 100 | 100 | 30 | 25 | 25 | 25 | 25 | 25 | 25 | 25 | 25 |

The above described data from the in vivo studies are consistent with the observations of the in vitro studies. The strain FI-6, which was sensitive to killing activity by MAb6D7 in vitro, was also sensitive in vivo, as evidenced by a significant increase in the survival of the mice treated with the monoclonal antibody. However, mice given both cefepime and MAb6D7 survived statistically significantly better than mice given either agent alone. The other strain, FI-3, in in vitro studies was relatively insensitive to killing by MAb6D7 without cefepime, and exhibited a modest increase of killing in the presence of the antibiotic. The in vivo results with FI-3 were consistent with this data in that there was no increase in survival of the mice treated with MAb6D7 without antibiotic. However, administration of both cefepime and 6D7 together caused a significant delay in the death of the FI-3 infected mice. These results are in contrast to those with the control antibody, in which killing was not significantly enhanced by the antibiotic.

The low dose of cefepime used in the above described studies is intended to mimic a clinical situation in which the amount of antibiotic which reaches the infection site is insufficient to cause bacterial killing.

Immunoblot Analysis of Whole Cell Lysates Using Monoclonal Antibodies 6D7 and 4A12

The identification of the bacterial antigen to which monoclonal antibodies 6D7 and 4A12 bind was determined by immunoblot analysis of whole cell lysates.

Preparation of the Cell Lysates

The method for preparing whole cell lysates is based on Hitchcock and Brown, J. Bacteriol. (1983), 154,269. More specifically, P. aeruginosa strains ATCC Accession Nos. 27312-27318, which are representatives of FI types 1-7, respectively, and E. coli strain 294 were grown to log phase in 10 ml of adjusted Mueller-Hinton Broth at 37°C. Cells were harvested by centrifugation at 600 rpm for 10 min at 4°C. The cell pellets were washed by resuspension in cold phosphate buffered saline (PBS), and centrifuged as described above. Cell pellets were resuspended in PBS to an $O.D._{660}$ of 0.40, lysozyme (Sigma Corp.) was added to a concentration of 1 mg/ml, and the suspensions were incubated at 37°C for 1 hr, and centrifuged as described above. The resulting cell pellets were resuspended in 0.5 ml buffer containing 0.625 M Tris HCl, pH 6.8, 10% sodium dodecyl sulfate (SDS), 20% glycerol, 5% beta-mercaptoethanol, and 0.5% bromphenol blue. The cell suspensions were heated for 10 min at 100°C, allowed to cool to 56°C, 10 $\mu$g/ml proteinase K (Boehringer Mannheim Biochemicals) was added, and the suspensions were incubated for 1 hr at 56°C. These samples were designated whole cell lysates.

Immunoblot Analysis of the Antigen to Which MAb6D7 Binds Using 12% Polyacrylamide Gels

The cell lysates were subjected to SDS-polyacrylamide gel electrophoresis (SDS-PAGE) using a Novex precast gel system (Novex, Encintas, CA). Each of the whole cell lysates (0.02 ml) was loaded into a lane in a 12% SDS gel (Novex cat. no. EC1005). Electrophoresis was for 1 hr at 36 mA/gel, using as buffer that described by Laemmli (Nature (1970), 227, 680). Electrophoretic transfer to nitrocellulose was at 200 mA for 1 hr according to the manufacturer's (Novex) directions, except that the transfer buffer contained 20% methanol, 25 mM Tris, 192 mM glycine, and 0.05% SDS. Immunological development of the nitrocellulose blot was performed using 50 $\mu$g/ml of affinity purified monoclonal antibody 6D7 in PBS/Tween. Detection of the 6D7 antibody-antigen complex was with alkaline phosphatase conjugated anti-mouse Ig (Tago, Burlingame, CA).

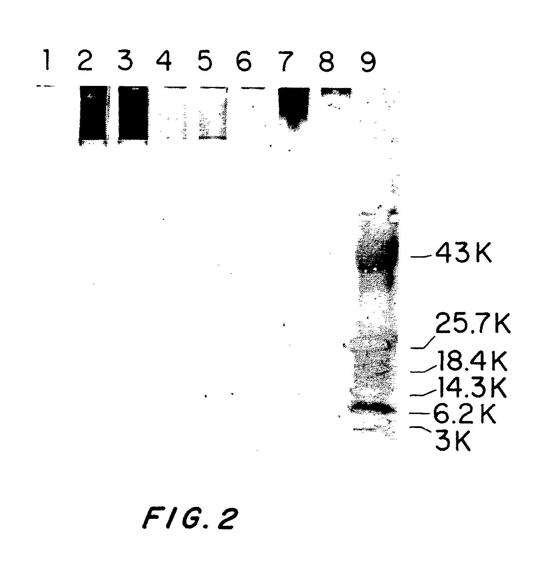

Photographs of the immunoblots are shown in Fig. 2. The lanes contained the following cell lysates: (1) E. coli strain 294; (2) P. aeruginosa FI-7; (3) P. aeruginosa FI-6; (4) P. aeruginosa FI-5; (5) P. aeruginosa FI-4; (6) P. aeruginosa FI-3; (7) P. aeruginosa FI-2; and (8) P. aeruginosa FI-1. Lane 9 of the gel contained molecular weight standards. As seen in Fig. 2, monoclonal antibody 6D7 bound to a slow migrating smear of antigen in each sample lane containing P. aeruginosa cell lysate. Binding was not observed in the lane containing the E. coli cell lysate. In addition, the antigenic determinant to which 6D7 bound was not affected by the extensive proteinase K treatment used in the preparation of the cell lysates; thus it appears to be of a carbohydrate nature. The molecular weight of the antigen, as indicated by the position of the smear, differentiates it from another common carbohydrate antigen found in P. aeruginosa, described in U.S. Patent No. 4,777,136, which migrates with an apparent molecular weight of 12 kD.

Immunoblot Analysis with MAb6D7 and MAb4A12

In order to determine if monoclonal antibody 6D7 (MAb6D7) and monoclonal antibody 4A12 (MAb4A12) bind to the same high molecular weight antigen, immunoblot analysis of whole cell P. aeruginosa lysates using MAb6D7 and MAb4A12 were performed.

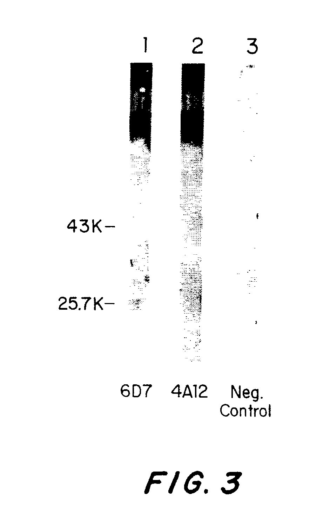

Whole cell lysates obtained from P. aeruginosa FI-1 were subjected to electrophoresis in a 4-20% SDS gel, and were transferred to nitrocellulose as described above. The paper was cut into sections, and the sections treated with either MAb6D7, MAb4A12, or a negative control antibody. Immunological staining and detection was as described above. An examination of the stained sections, shown in Fig. 3, showed that MAb6D7 and MAb4A12 were both bound to exactly the same region of the gel. Binding was not observed with the negative control antibody. Thus it appears that MAb6D7 and MAb4A12 bind to the same high molecular weight P. aeruginosa antigen.

Immunoblot Analysis with MAb6D7, MAb4A12, and anti-LPS Antibody

In order to ascertain that the antigen to which MAb4A12 and MAb6D7 binds is not LPS, immunoblot analysis of P. aeruginosa whole cell lysates using MAb6D7, MAb4A12, and anti-LPS antibody were performed.

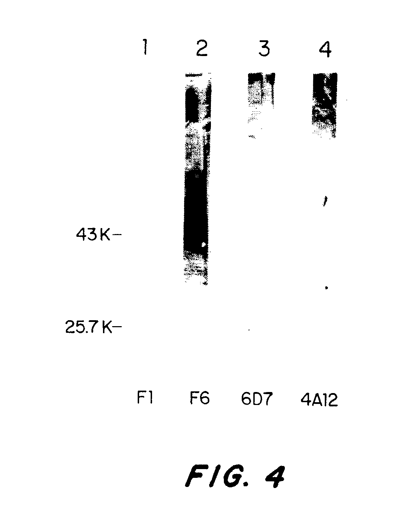

A whole cell lysate was prepared from P. Aeruginosa Fl-6, using essentially the technique described above, except that the Fl-6 strain was substituted for the Fl-1 strain. The lysate was subjected to electrophoresis and transfer onto nitrocellulose as described above for the Fl-1 whole cell lysates. After the transfer, the nitrocellulose paper was cut into sections, and treated with the monoclonal antibodies indicated in Fig. 4. Staining and detection of antigen-antibody complexes was as described above, using alkaline phosphatase conjugated anti-mouse Ig. A photograph of the stained sections is shown in Fig. 4. In the figure, the sections were treated with the following antibodies: lane 1, anti-LPS directed to P. aeruginosa Fl-1 LPS (negative control antibody); lane 2, anti-LPS directed to P. aeruginosa Fl-6 LPS (serotype specific antibody for the Fl-6 strain); lane 3, MAb6D7; and lane 4, MAb4A12.

As seen from Fig. 4, both MAb6D7 and MAb4A12 bind to the same region in the gel. This region is different than the one to which the anti-Fl-6 LPS binds. The negative control antibody did not bind. Thus, these results confirm the results with the Fl-1 lysate that MAb6D7 and MAb4A12 bind to the same antigen. In addition, they demonstrate that the antigen to which MAb6D7 and MAb4A12 bind is distinguishable from LPS.

Immunoblot Analysis Using a 4-12% Gradient Gel

Further characterization of the size of the antigen to which MAb6D7 binds was achieved by subjecting the whole cell lysates, prepared as described above, to electrophoresis on a 4-12% gradient gel. The conditions for electrophoresis, transfer, and immunodevelopment of the blots were essentially as described above, except that the gradient gel was substituted for the 12% gel.

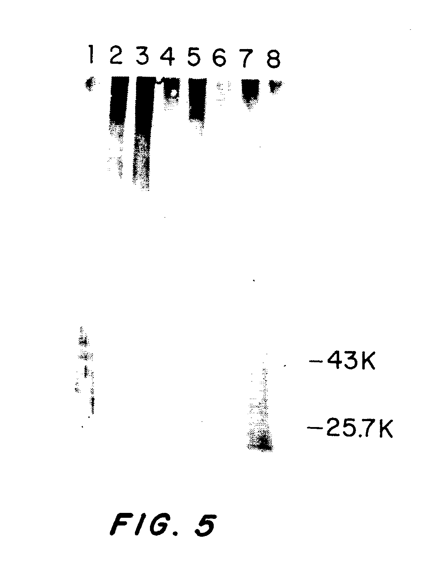

A photograph of the immunoblot is shown in Fig. 5. The lanes contained the following: (1) P. aeruginosa Fl-1 (outer membrane); (2) P. aeruginosa Fl-6 (lysate); (3) P. aeruginosa Fl-5 (lysate); (4) P. aeruginosa Fl-4 (lysate); (5) P. aeruginosa Fl-3 (lysate); (6) P. aeruginosa Fl-2 (lysate); and (7) P. aeruginosa Fl-1 (lysate). The position of the molecular weight standards is indicated. As seen from the photograph, the material from each cell lysate which bound the 6D7 monoclonal antibody had migrated as a high molecular weight smear. However, the change in acrylamide concentration allowed more resolution of the antigen. A series of bands between 25kD and 43 kD was observed in the outer membrane preparation and the lysates of several of the strains. It appeared from the results that the epitope which is recognized by 6D7 is on an antigen that is heterogeneous in size.

Distinguishing the Antigen to Which 6D7 Binds From A Band and B Band LPS

Currently there are two recognized LPS fractions in P. aeruginosa, designated A band and B band LPS (Lam et al. (1989), J. Clinical Microbiology, 27, 962). Although the B band LPS contains the highly immunogenic carbohydrate structures which define serotype specificity, it also contains at least one epitope which is common to all the LPS, and which is on a 12 Kd fraction. See U.S. Patent No. 4,777,136. The A band fraction of LPS also contains epitopes that are common to a wide variety of different P. aeruginosa strains. The determination that the 6D7 epitope was a common epitope on a unique cellular structure, i.e., was not on the A band or B band of LPS was as follows.

In order to ascertain whether the epitope recognized by monoclonal antibody produced by 6D7 was on the A band or the B band fraction of LPS, immunoblot analysis was performed on a preparation of outer membranes isolated from P. aeruginosa strain AK1401. Strain AK1401 is a mutant strain which is deficient in the B band fraction of LPS. Lam et al. (1989), J. Clinical Microbiol. 27, 962; Berry and Kropinski (1986), Can. J. Microbiol. 32, 436. The antibodies used in the immunoblot analysis were monoclonal antibody 6D7, and the anti-A band monoclonal antibody N1F10. The latter antibody is described in Lam et al. (1989), J. Clinical Microbiol. 27, 962. The outer membranes were prepared with sarkosyl, as described in Lambert et al. (1982), FEMS 14, 43. The membrane samples were subjected to immunoblot analysis as described supra, using the 6D7 and N1F10 antibodies.

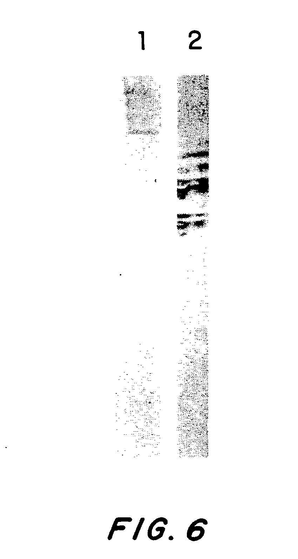

Photographs of the immunoblots of the outer membrane preparations of strain AK1401 are shown in Fig. 6, where lane 1 is an immunoblot using monoclonal antibody 6D7, and lane 2 is an immunoblot using monoclonal antibody N1F10. As seen in the figure, monoclonal antibody 6D7 was immunologically bound to material which migrated in the gel as a heterogeneous high molecular weight product. In contrast, monoclonal antibody N1F10 was bound to material of significantly lower molecular weight than that to which 6D7 was bound. Since monoclonal antibody N1F10 is specific for the A band of LPS, these results provide evidence that the epitope to which 6D7 binds is not located on the A band of the LPS fraction. Moreover, since strain AX1401 is deficient in B band LPS, but still immunologically binds 6D7, the results also indicate that the epitope to which 6D7 binds is on an antigen other than B band LPS. Therefore, it appears that the epitope to which 6D7 binds is one which has hitherto been undefined.

The following materials are on deposit under the terms of the Budapest Treaty with the American Type Culture Collection (ATCC), 12301 Parklawn Dr., Rockville, Maryland 20852, and have been assigned the following Accession Numbers.

|  | ATCC No. | Deposit Date |
|---|---|---|
| Murine hybridoma 4A12 | HB 10352 | Feb. 8, 1990 |
| Murine hybridoma 6D7 | HB 10353 | Feb. 8, 1990 |

The deposited materials mentioned herein are intended for convenience only, and are not required to practice the present invention in view of the descriptions herein. These materials are incorporated herein by reference.

Commercial Utility

The antibodies of the invention are useful for the treatment and diagnosis of P. aeruginosa infections in individuals. They are also useful for the detection of P. aeruginosa contamination of foodstuffs, medical instruments, and potable fluids, and water.

**Claims**

1. A composition comprising a first monoclonal antibody or binding fragment thereof which is capable of immunologically reacting with an antigen with which a second monoclonal antibody is capable of immunologically reacting, wherein the second monoclonal antibody is a monoclonal antibody produced by hybridoma 6D7 or hybridoma 4A12.

2. A composition according to Claim 1, wherein the first monoclonal antibody, or fragment, is capable of blocking the binding to an epitope with which a second monoclonal antibody binds, wherein the second monoclonal antibody is a monoclonal antibody produced by hybridoma 6D7 or 4A12.

3. A composition as claimed in Claim 2, wherein the monoclonal antibody is produced by hybridoma 6D7 or hybridoma 4A12.

4. A composition according to any one of Claims 1 to 3 which further comprises an antibiotic agent.

5. A pharmaceutical composition comprising a composition according to any of Claims 1 to 4 and a pharmaceutically acceptable carrier, diluent or excipient.

6. The monoclonal antibody produced by hybridoma 6D7, or an immunologically reactive fragment thereof.

7. The monoclonal antibody produced by hybridoma 4A12, or an immunologically reactive fragment thereof.

8. A cell line which produces an antibody capable of immunologically reacting with an antigen with which a monoclonal antibody produced by hybridoma 6D7 or hybridoma 4A12 is capable of immunologically reacting.

9. A cell line which produces an antibody capable of immunologically reacting with an epitope with which a monoclonal antibody produced by hybridoma 6D7 or hybridoma 4A12 is capable of immunologically reacting.

10. A cell line having the identifying characteristics of hybridoma 4A12 or hybridoma 6D7.

11. Hybridoma cell line 4A12.

12. Hybridoma cell line 6D7.

13. A process for producing an antibody specific for an antigen of P. aeruginosa, which comprises cultivating a cell line of any one of Claims 8 to 12 and, if desired, recovering an antibody produced.

14. An antigen which is immunologically reactive with a monoclonal antibody produced by hybridoma 6D7 or 4A12.

15. The antigenic determinant which is immunologically reactive with a monoclonal antibody produced by hybridoma 6D7 or 4A12.

16. An antibody, or fragment thereof, which is reactive with the antigen of Claim 14.

17. An antibody, or fragment thereof, which is reactive with the antigenic determinant of Claim 15.

18. An antibody as claimed in Claim 16 or 17 which is a monoclonal antibody, or immunologically reactive fragment thereof.

19. An antibody as defined in any one of Claims 1 to 7 or 16 to 18 which is a hybrid, chimeric, altered, univalent, or single domain antibody; or, a fragment thereof.

20. A hybridoma cell which produces an antibody as claimed in Claim 18.

21. A kit-of-parts adapted for the treatment, prevention or diagnosis of a P. aeruginosa infection which comprises an antibody, or fragment thereof, as defined in any one of Claims 1 to 7 or 16 to 19.

22. An antibody or fragment thereof, as defined in any one of Claims 1 to 7 or 16 to 19, for use in the treatment, prevention, or diagnosis of infection.

23. A method for detecting a P. aeruginosa infection which comprises:
a. providing a sample in which a P. aeruginosa antigen is to be detected;
b. interacting said sample with an antibody, or fragment thereof, as defined according to any one of Claims 1 to 7 or 16 to 19, under conditions which allow an immunological reaction between the antigen of (a) and the composition;
c. detecting a complex formed between the composition of (b) and the antigen of (a).

**Claims for the following Contracting State: GR**

1. A process for producing an antibody specific for an antigen of P. aeruginosa, which comprises cultivating a cell line which produces a first monoclonal antibody which is capable of immunologically reacting with an antigen with which a second monoclonal antibody is capable of immunologically reacting, wherein the second monoclonal antibody is a monoclonal antibody produced by hybridoma 6D7 or hybridoma 4A12 and, if desired, recovering an antibody produced, and further, if desired, preparing an immunologically reactive fragment of the first antibody.

2. A process as claimed in Claim 1, wherein the first monoclonal antibody, or fragment, is capable of blocking the binding to an epitope with which a second monoclonal antibody binds, wherein the second monoclonal antibody is a monoclonal antibody produced by hybridoma 6D7 or 4A12.

EP 0 450 573 A2

3. A process as claimed in Claim 2, wherein the cell line cultivated is hybridoma 6D7 or hybridoma 4A12.

4. A process as claimed in any one Claims 1 to 4 in which the antibody produced is MAb6d7 or MAb4A12.

5. A process for preparing a pharmaceutical formulation which comprises admixing with a pharmaceutically acceptable carrier, diluent or excipient a monoclonal antibody, or binding fragment thereof, which is capable of immunologically reacting with an antigen with which a second monoclonal antibody is capable of immunologically reacting, wherein the second monoclonal antibody is a monoclonal antibody produced by hybridoma 6D7 or hybridoma 4A12.

6. A process as claimed in Claim 5 in which the first monoclonal antibody, or fragment thereof, is capable of blocking the binding to an epitope with which a second monoclonal antibody binds, wherein the second monoclonal antibody is produced by hybridoma 6D7 or 4A12.

7. A process as claimed in Claim 5 or 6 in which the monoclonal antibody is produced by hybridoma 6D7 or hybridoma 4A12.

8. A process as claimed in any one Claims 5 to 7 which further comprises adding an antibiotic agent to the formulation.

9. A process as claimed in any one of Claims 1 to 8 in which the antibody is a hybrid, chimeric, altered, univalent, or single domain antibody; or, a fragment thereof.

10. A method for detecting a P. aeruginosa infection which comprises:

   a. providing a sample in which a P. aeruginosa antigen is to be detected;

   b. interacting said sample with an antibody, or fragment thereof, as defined in any one of Claims 1 to 9, under conditions which allow an immunological reaction between the antigen of (a) and the composition;

   c. detecting a complex formed between the composition of (b) and the antigen of (a).

11. A kit-of-parts adapted for the treatment, prevention or diagnosis of a P. aeruginosa infection which comprises an antibody, or fragment thereof, as defined in any one of Claims 1 to 9.

12. A cell line which produces an antibody capable of immunologically reacting with an antigen with which a monoclonal antibody produced by hybridoma 6D7 or hybridoma 4A12 is capable of immunologically reacting.

11. A cell line which produces an antibody capable of immunologically reacting with an epitope with which a monoclonal antibody produced by hybridoma 6D7 or hybridoma 4A12 is capable of immunologically reacting.

12. A cell line having the identifying characteristics of hybridoma 4A12 or hybridoma 6D7.

13. Hybridoma 4A12 or 6D7.

14. An antigen which is immunologically reactive with a monoclonal antibody produced by hybridoma 6D7 or 4A12.

15. The antigenic determinant which is immunologically reactive with a monoclonal antibody produced by hybridoma 6D7 or 4A12.

16. The monoclonal antibody produced by hybridoma 6D7, or an immunologically reactive fragment thereof.

17. The monoclonal antibody produced by hybridoma 4A12, or an immunologically reactive fragment thereof.

18. The antigenic determinant which is immunologically reactive with a monoclonal antibody produced by hybridoma 6D7 or 4A12.

19. An antibody, or fragment thereof, which is reactive with the antigen of Claim 14.

20. An antibody, or fragment thereof, which is reactive with the antigenic determinant of Claim 15.

21. An antibody as claimed in Claim 19 or 20 which is a monoclonal antibody, or immunologically reactive fragment thereof.

22. An antibody as defined in any one of Claims 1 to 4 or 19 to 21 which is a hybrid, chimeric, altered, univalent, or single domain antibody; or, a fragment thereof.

23. A hybridoma cell which produces an antibody as claimed in Claim 21.


**Claims for the following Contracting State: ES**


1. A process for producing an antibody specific for an antigen of P. aeruginosa, which comprises cultivating a cell line which produces a first monoclonal antibody which is capable of immunologically reacting with an antigen with which a second monoclonal antibody is capable of immunologically reacting, wherein the second monoclonal antibody is a monoclonal antibody produced by hybridoma

20

EP 0 450 573 A2

6D7 or hybridoma 4A12 and, if desired, recovering an antibody produced, and further, if desired, preparing an immunologically reactive fragment of the first antibody.

2. A process as claimed in Claim 1, wherein the first monoclonal antibody, or fragment, is capable of blocking the binding to an epitope with which a second monoclonal antibody binds, wherein the second monoclonal antibody is a monoclonal antibody produced by hybridoma 6D7 or 4A12.

3. A process as claimed in Claim 2, wherein the cell line cultivated is hybridoma 6D7 or hybridoma 4A12.

4. A process as claimed in any one Claims 1 to 4 in which the antibody produced is MAb6d7 or MAb4A12.

5. A process for preparing a pharmaceutical formulation which comprises admixing with a pharmaceutically acceptable carrier, diluent or excipient a monoclonal antibody, or binding fragment thereof, which is capable of immunologically reacting with an antigen with which a second monoclonal antibody is capable of immunologically reacting, wherein the second monoclonal antibody is a monoclonal antibody produced by hybridoma 6D7 or hybridoma 4A12.

6. A process as claimed in Claim 5 in which the first monoclonal antibody, or fragment thereof, is capable of blocking the binding to an epitope with which a second monoclonal antibody binds, wherein the second monoclonal antibody is produced by hybridoma 6D7 or 4A12.

7. A process as claimed in Claim 5 or 6 in which the monoclonal antibody is produced by hybridoma 6D7 or hybridoma 4A12.

8. A process as claimed in any one Claims 5 to 7 which further comprises adding an antibiotic agent to the formulation.

9. A process as claimed in any one of Claims 1 to 8 in which the antibody is a hybrid, chimeric, altered, univalent, or single domain antibody; or, a fragment thereof.

10. A method for detecting a P. aeruginosa infection which comprises:
    a. providing a sample in which a P. aeruginosa antigen is to be detected;
    b. interacting said sample with an antibody, or fragment thereof, as defined in any one of Claims 1 to 9, under conditions which allow an immunological reaction between the antigen of (a) and the composition;
    c. detecting a complex formed between the composition of (b) and the antigen of (a).

11. A process for preparing a kit-of-parts adapted for the treatment, prevention or diagnosis of a P. aeruginosa infection which comprises incorporating into the kit-of-parts an antibody, or fragment thereof, as defined in any one of Claims 1 to 9.

12. A process for producing a cell line which produces an antibody capable of immunologically reacting with an antigen with which a monoclonal antibody produced by hybridoma 6D7 or hybridoma 4A12 is capable of immunologically reacting which comprises
    a. immunizing an individual with P. aeruginosa;
    b. immortalizing antibody producing cells from the immunized individual;
    c. selecting an immortal cell which produces antibodies which react immunologically with the antigen to which monoclonal antibodies produced by hybridoma 6D7 or hybridoma 4A12 react immunologically; and
    d. growing said immortal cells.

13. A process as claimed in claim 12 wherein the cell line produces an antibody capable of immunologically reacting with an epitope with which a monoclonal antibody produced by hybridoma 6D7 or hybridoma 4A12 is capable of immunologically reacting.

14. A process as claimed in claim 12, wherein the cell line has the identifying characteristics of hybridoma 4A12 or hybridoma 6D7.

15. A process as claimed in claim 12, wherein the cell line is hybridoma 4A12 or 6D7.

Fig. 1  Opsonophagocytic Assay with Pseudomonas aeruginosa F6
Effect of Antibody Concentration

1 2 3 4 5 6 7 8 9

—43K

25.7K

18.4K

14.3K

6.2K

3K

FIG.2

43K-

25.7K-

1    2    3

6D7   4A12   Neg.
Control

FIG. 3

FIG. 4

1 2 3 4 5 6 7 8

−43K

−25.7K

# FIG. 5

FIG. 6